# EUROPEAN PATENT APPLICATION

(11) **EP 1 142 874 A2**
(43) Date of publication of application: **10.10.2001**
(21) Application number: 01111214.1
(22) Date of filing: 24.11.1998
(51) Int. Cl.: C07D 209/88

(54) **Intermediates for preparing the R or S enantiomer of 1-[9'H-carbazol-4'-yloxy]-3-[ 2''-(2'''- methoxy -phenoxy)-ethyl -amino]-propan-2-ol[carvedilol]**

(30) Priority: 24.11.1997 HU 9702209; 01.10.1998 HU 9802180
(62) Divisional of application: 98122114.6
(71) Applicant: EGIS GYOGYSZERGYAR RT., 1106 Budapest (HU)
(72) Inventor: Ratkai, Zoltan, 1101 Budapest (HU); Barkoczy, Jozsef, Dr., 1016 Budapest (HU); Simig, Gyula, Dr., 1126 Budapest (HU); Gregor, Tamas, Dr., Csömör (HU); Vereczkey, Györgyi Donath, 1036 Budapest (HU); Németh, Norbert, 1119 Budapest (HU); Nagy, Kalman, Dr., 1025 Budapest (HU); Cselenyak, Judit, 1124 Budapest (HU); Szabo, Tibor, 1144 Budapest (HU); Balazs, Laszlo, 1088 Budapest (HU); Doman, Imre, 1035 Budapest (HU); Greff, Zoltan, Dr., 1028 Budapest (HU); Nagy, Péter Kotay, Dr., 2600 Vac (HU); Seres, Péter, Dr., 1053 Budapest (HU)
(74) Representative: Beszédes, Stephan G., Dr.

(57) **Abstract**

The subject matter of the invention are R-(+)-1-[N--{benzyl}-2'-({2''-<methoxy>-phenoxy)-ethyl}-amino]-3--[9'''H-carbazol-4'''-yloxy]-propan-2-ol and S-(-)-1-[N--{benzyl}-2'-({2''-<methoxy>-phenoxy)-ethyl}-amino]-3--[9'''H-carbazol-4'''-yloxy]-propan-2-ol and acid addition salts thereof.

Via these intermediates the end products R and S enantiomers of 1-[9'H-carbazol-4'-yloxy]-3-[{2''-(2'''--<methoxy>-phenoxy)-ethyl}-amino]-propan-2-ol [carvedilol] can be prepared in a very high purity simply and economically in high yields.

## Description

The present invention is concerned with new intermediates for preparing the optically active R or S enantiomer of 1-[9'H-carbazol-4'-yloxy]-3--[{2''-(2'''-<methoxy>-phenoxy)-ethyl}-amino]-propan-2-ol [carvedilol] of formula 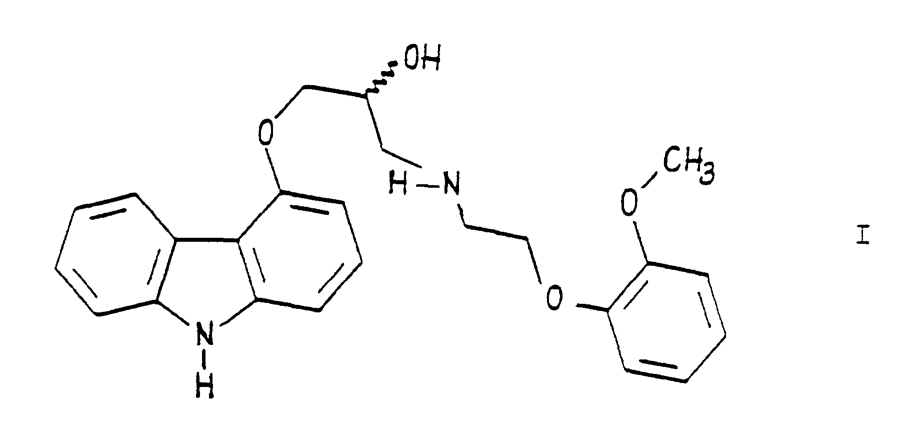 as well as mixtures of the enantiomers and acid addition salts of these compounds.

This compound is known under the INN name carvedilol and is used as a drug having antihypertensive, beta-adrenergic blocking and vasodilating activity. The chemical name thereof is as beforesaid, 1-[9'H-carbazol-4'-yloxy]-3-[{2''-(2'''--<methoxy>-phenoxy)-ethyl}-amino]-propan-2-ol.

The preparation of 1-[9'H-carbazol-4'-yloxy]-3-[{2"-(2"'--<methoxy>-phenoxy)-ethyl}-amino]-propan-2-ol [carvedilol] has been known from DE-OS No. 28 15 926, while the preparation of the R and S enantiomers has been described in DE-OS No. 33 19 027. According to the known process, 4-(oxiranylmethoxy)--9H-carbazole of formula 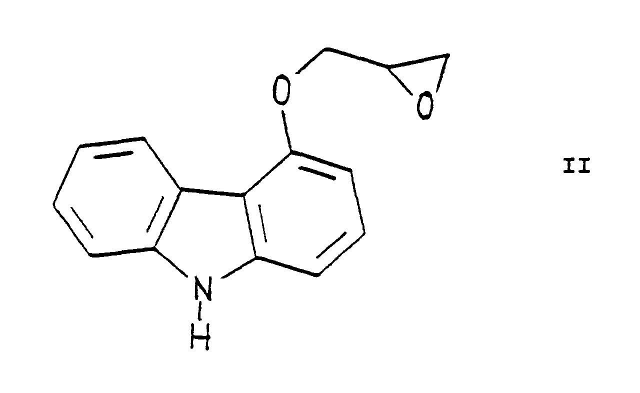 or the R or S enantiomer thereof is reacted with 2-[2'-- (methoxy)-phenoxy]-ethylamine of formula 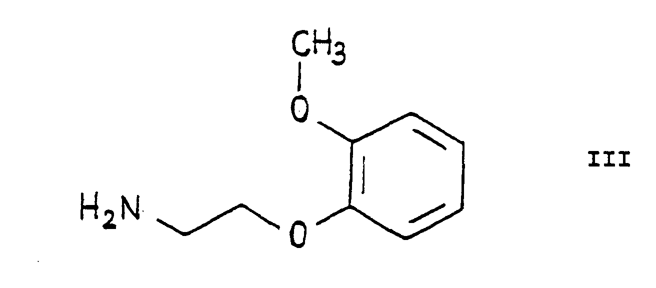 to produce the compound 1-[9'H-carbazol-4'-yloxy]-3-[{2''-- (2'''-<methoxy>-phenoxy)-ethyl}-amino]-propan-2-ol [carvedilol] of formula I in a yield of 39 to 42%. The drawback of the known process consists in the fact that parallel with the formation of 1-[9'H-carbazol-4'-yloxy]-3-[{2"-(2"'--<methoxy>-phenoxy)-ethyl}-amino]-propan-2-ol [carvedilol] also a bis compound of formula 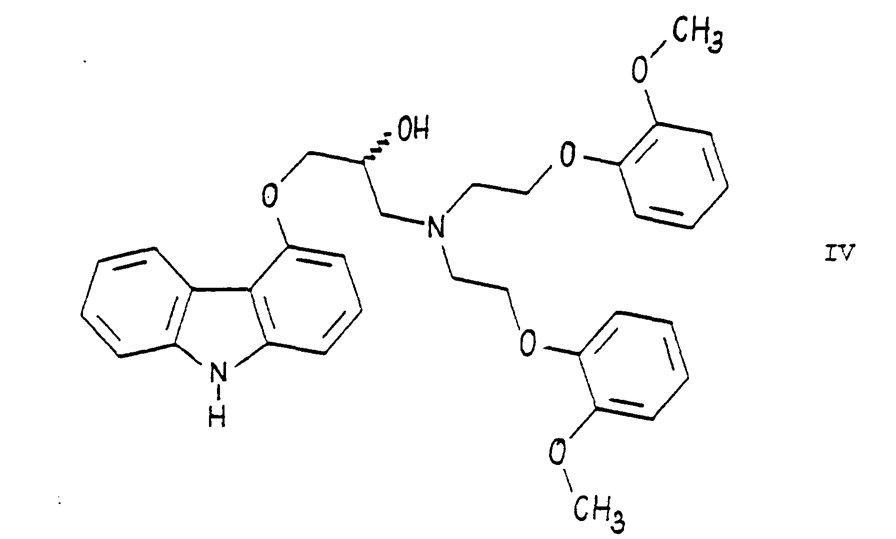 forms through the reaction of 2 molar equivalents of 4-(oxiranylmethoxy)-9-H-carbazole of formula II and 1 molar equivalent of 2-[2'-(methoxy)-phenoxy]-ethylamine of formula III. This side-reaction can never be avoided, and the bis compound of formula IV is formed in an amount that is commensurable with that of 1-[9'H-carbazol-4'-yloxy]-3-[{2''-- (2"'-<methoxy>-phenoxy)-ethyl}-amino]-propan-2-ol [carvedilol]. Consequently, the known process is uneconomical.

Also further possibilities for the preparation of [carvedilol] have been described in DE-OS No. 28 15 926. According to these processes, 4-[3'-(amino)-2'-(hydroxy)--propoxy]-9H-carbazole can be reacted with
a) 2- [2' - (methoxy) -phenoxy] -ethylhalide or -sulfonate;
b) 2-[2'-(methoxy)-phenoxy]-acetaldehyde followed by catalytic hydrogenation;
c) 2- [2' - (methoxy) -phenoxy] -acetylchloride, followed by reducing the formed acid amide with a complex metal hydride.

None of the processes a) to c) is suitable for the economical preparation of 1-[9'H-carbazol-4'-yloxy]-3-[{2''--(2'''-<methoxy>-phenoxy)-ethyl}-amino]-propan-2-ol [carvedilol]. In case of each process, a purification step by column chromatography is required for the separation of the product, and the preparation of the starting compound is uneconomical. In process a), the bis compound of formula IV forms, too. In case of process b), a yield of 41%, while in case of process c), a yield of 24% can be obtained, at the most. In process c), the use of a complex metal hydride, practically lithium aluminium hydride, is also a drawback since this reaction is accompanied by an enhanced fire hazard. The latter reaction requires absolute conditions, i.e. even traces of water should be avoided.

In principle, the formation of the bis compound of formula IV can be avoided by using, instead of the primary amine 2-[2'--(methoxy)-phenoxy]-ethylamine of formula III, a secondary amine which is a derivative of the primary amine 2-[2'-(methoxy)--phenoxy]-ethylamine of formula III containing a protective group. Such a secondary amine is, for example, the N-[2-(2'--<methoxy>-phenoxy)-ethyl]-benzylamine of formula

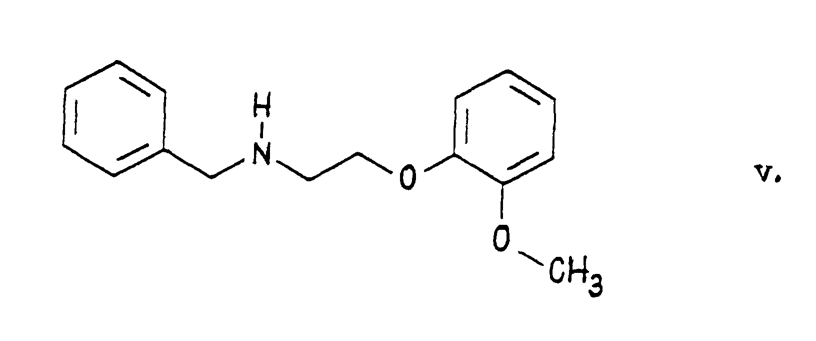

In Example 5 of DE-OS No. 28 15 926, the 4-(oxiranylmethoxy)--9H-carbazole of formula II is reacted with the secondary amine N-[2-(2'-<methoxy>-phenoxy)-ethyl]-benzylamine of formula V to yield the 1-[N-{benzyl}-2'-({2''-<methoxy>-phenoxy)ethyl}--amino]-3-[9'''H-carbazol-4'''-yloxy]-propan-2-ol [benzyl-carvedilol] of the formula 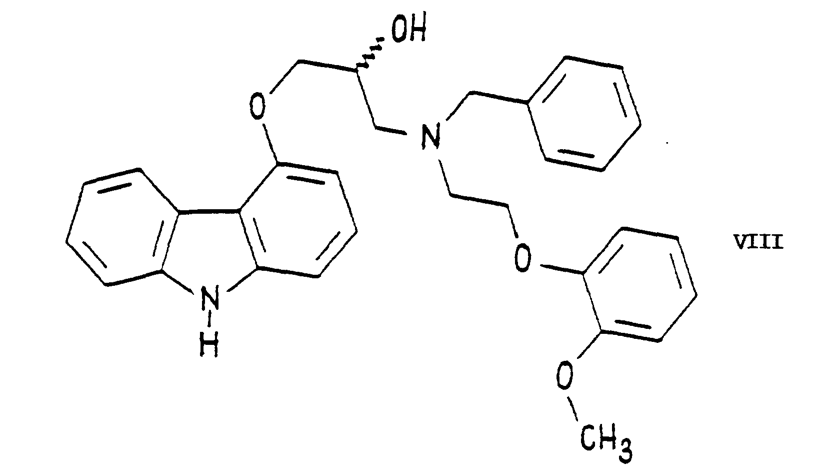 which could be separated only after a purification step by column chromatography. This procedure is uneconomical for manufacturing on an industrial scale. The reaction of the compounds of the formulae II and V was carried out in ethylene glycol dimethyl ether.

Example 5 of DE-OS No. 28 15 926 was reproduced on a fourfold scale. As shown by Example for Comparison No. I, only a yield of 60% could be achieved, and a contaminated product was obtained which has a melting point of 92°C significantly lower than the value indicated in Example 5 of DE-OS No. 28 15 926 (97 to 99°C), Without purification by column chromatography, it was failed to crystallize the product, thus, this purification procedure cannot be avoided. This is a considerable disadvantage in case of industrial application. Since the enlargement of scale of the above known process is accompanied by the reduction of both yield and quality of the product, said process is not suitable as an industrial one.

The process indicated in Example 5 of DE-OS No. 28 15 926 was modified by using, instead of ethylene glycol dimethyl ether, ethyl acetate or dioxan as shown by Examples for Comparison Nos. II and III. Even after a reaction period of 28 hours, about half of the starting compound remained unreacted in dioxan, and practically no reaction has taken place in ethyl acetate.

The problem underlying to the invention is to provide for new intermediates for preparing the optically active R or S enantiomer of 1-[9'H-carbazol-4'-yloxy]-3-[{2"-(2"'--<methoxy>-phenoxy)-ethyl}-amino]-propan-2-ol [carvedilol] by which by overcoming the above disdvantages of the known processes and intermediates this compound can be obtained with high yields and in a simple way with drastically reduced formation of byproducts and thus without the necessity of purification steps, which process also can be carried out on large scale industrially.

Surprisingly this has been achieved by the present invention.

The subject matter of the present invention are the new compounds R-(+)-1-[N-{benzyl}-2'-({2''-<methoxy>--phenoxy) -ethyl}-amino] -3-[9'''H-carbazol-4'''-yloxy]--propan-2-ol and S-(-)-[N-{benzyl}-2'-({2"-<methoxy>--phenoxy) -ethyl}-amino] -3- [9'''H-carbazol-4'''-yloxy]--propan-2-ol, and acid addition salts thereof, preferably the pharmaceutically acceptable ones, which are valuable intermediates via which the end product 1-[9'H-carbazol-4'--yloxy]-3- [{2''-(2'''-<methoxy>-phenoxy)-ethyl}-amino] -propan--2-ol [carvedilol] can be prepared by a process having orginality in a superior way.

These optically active enantiomers of 1-[N-{benzyl}-2'-({2''-<methoxy>--phenoxy)-ethyl}-amino]-3-[9"'H-carbazol-4'''-yloxy]--propan-2-ol [benzyl-carvedilol] of formula 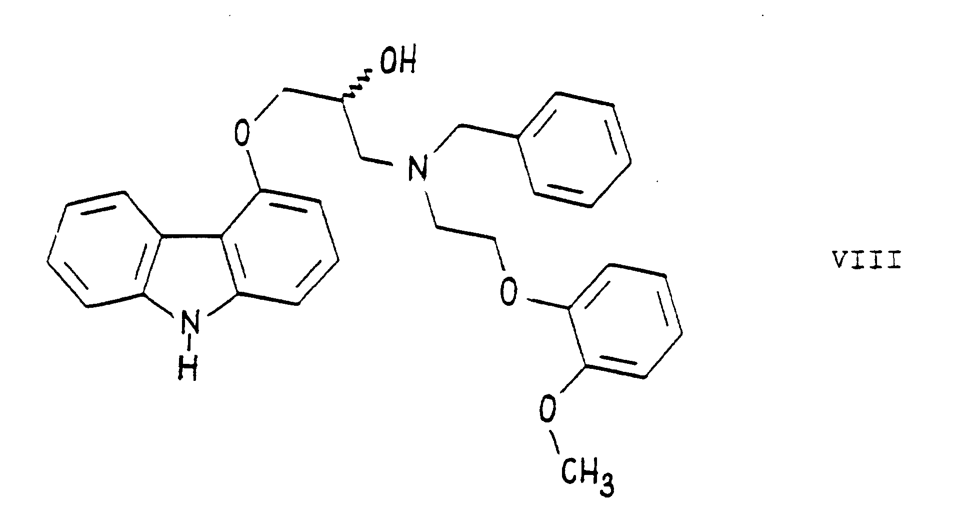 are novel compounds.

These intermediates can be prepared in the way that the R or S enantiomer of 4-(oxiranylmethoxy)-9H-carbazole of formula 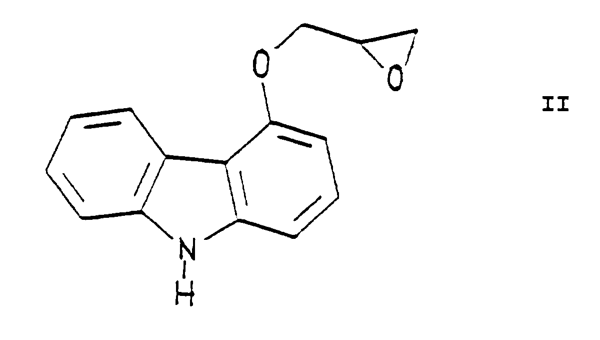 is reacted with the secondary amine N-[2-(2'-<methoxy>-phenoxy)-ethyl]--benzylamine of formula 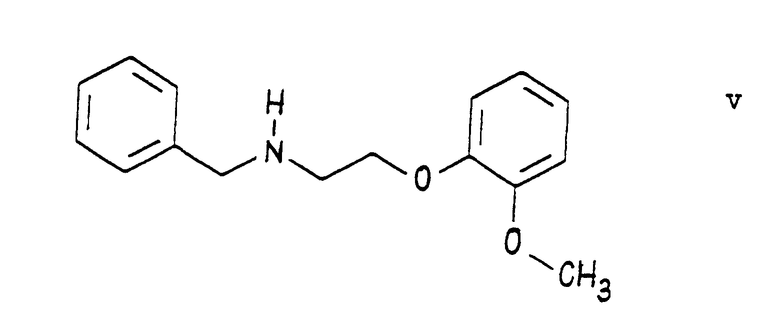 in a protic organic solvent, to yield the R or S enantiomer of 1-[N-{benzyl}-2'-({2''-<methoxy>-phenoxy)-ethyl}--amino]-3-[9'''H-carbazol-4'''-yloxy]-propan-2-ol [benzyl-carvedilol] of formula VIII, i. e. R-(+)-1-[N-{benzyl}-2'-({2''-<methoxy>-phenoxy)--ethyl}-amino]-3-[9'''H-carbazol-4'''-yloxy]-propan--2-ol or S-(-)-1-[N-{benzyl}-2'-({2''-<methoxy>--phenoxy)-ethyl}-amino]-3-[9'''H-carbazol-4'''-yloxy]--propan-2-ol and acid addition salts thereof.

These intermediates can be converted into their acid addition salts in a manner known per se.

From the thus formed R and S enantiomers of 1-[N-{benzyl}-2'-({2''-<methoxy>-phenoxy)-ethyl}-amino]--3-[9'''H-carbazol-4'''-yloxy]-propan-2-ol [benzyl-carvedilol] of formula VIII the end products R and S enantiomers of 1-[9'H-carbazol-4'-yloxy]-3-[{2''-(2'''--<methoxy>-phenoxy)-ethyl}-amino]-propan-2-ol [carvedilol] of formula I can be prepared by debenzylation by catalytic hydrogenation and, in a manner known per se, if desired, the R and S enantiomers 1-[9'H-carbazol-4'-yloxy]-3-[{2''-(2'''--<methoxy>-phenoxy)-ethyl}-amino]-propan-2-ol thus obtained can be reacted with inorganic or organic acids to yield acid addition salts thereof or, if desired, the free 1-[9'H--carbazol-4'-yloxy]-3-[{2''-(2'''-<methoxy>-phenoxy)-ethyl}--amino]-propan-2-ol base [carvedilol] of formula I from acid addition salts thereof can be liberated and, if desired, the free 1-[9'H-carbazol-4'-yloxyl-3-[{2"-(2"'-<methoxy>--phenoxy)-ethyl}-amino]-propan-2-ol base [carvedilol] can be converted into other acid addition salts and/or, if desired, separating the enantiomers.

In the above process the protic solvent is preferably 1 or more C₁₋₄ alkanol(s), particularly ethanol and/or isopropanol, most particularly the second one, and the reaction of the 4-(oxiranylmethoxy)-9H-carbazole of formula II with the secondary amine N-[2-(2'-<methoxy>--phenoxy)-ethyl]-benzylamine of formula V is carried out advantageously at 0 bis 120°C, preferably at the boiling point of the reaction mixture.

The secondary amine N-[2-(2'-<methoxy>-phenoxy)-ethyl]-benzylamine of formula V can be employed as an oil, i.e. in a form without crystal water, but preferably in a foxm containing crystal water.

The formed 1-[N-{benzyl}-2'-({2''-<methoxy>--phenoxy)-ethyl}-amino]-3-[9'''H-carbazol-4'''-yloxy]--propan-2-ol [benzyl-carvedilol] of formula VIII is either separated and then debenzylated, or preferably it is not separated from the reaction mixture in which it was prepared before the debenzylation reaction. The debenzylation by catalytic hydrogenation can be carried out in a manner known per se in connection with the removal of a benzyl group.

Preferably as a catalyst palladium on carbon is used. The catalyst can be used several times, without regeneration procedure, for the debenzylation of the 1-[N-{benzyl}-2'-({2''--<methoxy>-phenoxy)-ethyl}-amino]-3-[9"'H-carbazol-4''' -yloxy]-propan-2-ol [benzyl-carvedilol] of formula VIII. Preferably the hydrogenation is carried out using hydrazine hydrate.

The R and S enantiomers of the 4-(oxiranylmethoxy)-9H--carbazole of formula II usable as starting compounds can have been prepared by the process known from DE-OS No. 33 19 027. The secondary amine N-[2-(2'-<methoxy>-phenoxy)-ethyl]--benzylamine of formula V can have been prepared by the method of Augstein (J. Med. Chem., 8 [1965], 356 to 367) or in the manner given in the description.

The R and S enantiomers of 1-[N-{benzyl}-2'-({2''--<methoxy>-phenoxy)-ethyl}-amino]-3-[9'''H-carbazol-4'''--yloxy]-propan-2-ol [benzyl-carvedilol] of formula VIII are obtained in a very pure state, and consequently no separation is required, and they can be debenzylated in the reaction mixture in which they were formed.

Also the R and S enantiomers of 1-[9'H-carbazol-4'--yloxy]-3-({2''-(2'''-<methoxy>-phenoxy)-ethyl}-amino]--propan-2-ol [carvedilol] can be obtained in a very high purity in a yield of about 80%. In the process no bis compound of formula IV forms.

On the basis of the above characteristics, the above process can be carried out simply and economically.

An expert could not expect that, by using a protic organic solvent, the reaction of the R or S enantiomer of 4-(oxiranylmethoxy)-9H-carbazole of formula II and the secondary amine N-[2-(2'-<methoxy>-phenoxy)-ethyl]--benzylamine of formula V gives, without special purification procedure the R or S enantiomer of 1-[N-{benzyl}-2'-({2''--<methoxy>-phenoxy)-ethyl}-amino]-3-[9'''H-carbazol-4'''--yloxy]-propan-2-ol [benzyl-carvedilol] of formula VIII in a well crystallizing and pure form and in a yield of near or above 90%, since, on the basis of the Examples for Comparison, with the opposite of it could be reckoned.

The invention is further elucidated by means of the following Examples.

### Example 1

### S-(-)-1-[N-{Benzyl}-2'-({2''-<methoxy>-phenoxy)-ethyl}--amino]-3-[9'''H-carbazol-4'''-yloxy]-propan-2-ol

To 25 ml of isopropanol 3.85 g (13.1 mmoles) of N-[2-(2'--<methoxy>-phenoxy)-ethyl]-benzylamine and 2.4 g (10.0 mmoles) of S-(+)-4-(oxiranylmethoxy)-9H-carbazole are added, the reaction mixture is boiled for 7 hours under stirring, and the mixture is evaporated under reduced pressure. On standing, the evaporation residue crystallizes, the crystal mass is stirred with 12 ml of diethyl ether for half an hour, the crystals are filtered and washed twice using 10 ml of diethyl ether each time. The crude product is recrystallized from 40 ml of diisopropyl ether.

Thus, 3.52 g (70.6%) of the title compound S-(-)-1-- [N-{benzyl}-2'-({2"-<methoxy>-phenoxy)-ethyl}-amino]-3--[9'''H-carbazol-4'''-yloxy]-propan-2-ol are obtained. M.p.: 102.5 to 103.5°C. [α]_{D} = -9.6° (20°C, c = 1, acetic acid). [α]_{435 nm} = -14.5° (20°C, c = 1, acetic acid).

### Example 2

### R- (+) -1- [N-{Benzyl}-2'-({2''-<methoxy>-phenoxy)--ethyl}-amino]-3-[9'''H-carbazol-4"'-yloxy]-propan--2-ol

To 25 ml of isopropanol, 3.85 g (13.1 mmoles) of N-[2-(2'--<methoxy>-phenoxy)-ethyl]-benzylamine and 2.4 g (10.0 mmoles) of R-(-)-4-(oxiranylmethoxy)-9H-carbazole are added, the reaction mixture is boiled for 7 hours under stirring, and the mixture is evaporated under reduced pressure. The evaporation residue is crystallized from diisopropyl ether, and the crystals are filtered. The crude product is suspended in 50 ml of diisopropyl ether, the suspension is heated to boiling, then cooled and filtered.

Thus, 4.09 g (82.1% of the title compound R-(+)-1-- [N-{benzyl}-2'-({2''-<methoxy>-phenoxy)-ethyl}-amino]-3-- [9'''H-carbazol-4'''-yloxy]-propan-2-ol are obtained. M.p.: 103 to 104°C. [α]_{D} = +8.2° (20°C, c = 1, acetic acid). [α]_{435 nm} = +12.4° (20°C, c = 1, acetic acid).

### Example 3

### R-(+)-1-[9'H-Carbazol-4'-yloxy]-3-[{2"-(2"'-<methoxy>--phenoxy)-ethyl}-amino]-propan-2-ol

To 40 ml of ethanol, 1.99 g (4 mmoles) of R-(+)-1-- [N-{benzyl}-2'-({2''-<methoxy>-phenoxy)-ethyl}-amino]-3--[9'''H-carbazol-4'''-yloxy] -propan-2-ol, prepared as described in Example 2, and 1.0 g of wet palladium on carbon catalyst having a water content of 50.6% by weight and containing 16.2% by weight of palladium calculated on the dry catalyst are added. To the mixture 4 ml (80 mmoles) of 98% by weight hydrazine hydrate are added, drop by drop, at 20 to 30°C in 20 minutes, and the reaction mixture is stirred at 25°C for 1.5 hours. The catalyst is filtered off, washed twice with ethanol using 30 ml of ethanol each time, and the filtrate is evaporated under reduced pressure. To the evaporation residue, 80 ml of water and 80 ml of 1,2-dichloromethane are added, the mixture is stirred until the evaporation residue is dissolved, then the phases are separated, and the aqueous phase is extracted still twice using 80 ml of 1,2-dichloromethane each time. The organic phase is dried, then evaporated under reduced pressure. The evaporation residue is heated to boiling with 5 ml of ethyl acetate, the mixture is filtered, while hot, through a folded paper filter, and the filtrate is allowed to cool under stirring. After the precipitation of the crystals, the mixture is stirred at 25°C for further 8 hours, the crystals are filtered off, and washed twice using 3 ml of cold ethyl acetate each time. The crude product is recrystallized from 8 ml of ethyl acetate.

Thus, 1.16 g (71.2%) of the title compound R-(+)-1-[9'H--carbazol-4'-yloxy]-3-[{2''-(2'''-<methoxy>-phenoxy)-ethyl}--amino]-propan-2-ol are obtained as white crystals. M.p.: 112 to 113°C.
[α]_{D} = +17.2° (20°C, c = 1, acetic acid).

### Example 4

### S-(-)-1-[9'H-Carbazol-4'-yloxy]-3-[{2''-(2'''-<methoxy>--phenoxy)-ethyl}-amino]-propan-2-ol

To 35 ml of ethanol, 1.74 g (3.5 mmoles) of S-(-)-1--[N-{benzyl}-2'-({2''-<methoxy>-phenoxy)-ethyl}-amino]-3-- [9'''H-carbazol-4'''-yloxy]-propan-2-ol, prepared as described in Example 1, and 0.9 g of wet palladium on carbon catalyst having a water content of 50.6% by weight and containing 16.2% by weight of palladium calculated on the dry catalyst are added. To the mixture 3.5 ml (70 mmoles) of 98% by weight hydrazine hydrate are added, drop by drop, at 20 to 30°C in 10 minutes, and the reaction mixture is stirred at 25°C for 1.5 hours. The catalyst is filtered off, washed twice with ethanol using 30 ml of ethanol each time, and the filtrate is evaporated under reduced pressure. To the evaporation residue, 70 ml of water and 70 ml of 1,2-dichloromethane are added, the mixture is stirred until the evaporation residue is dissolved, then the phases are separated, and the aqueous phase is still twice extracted using 70 ml of dichloromethane each time. The organic phase is dried, then evaporated under reduced pressure. The evaporation residue is heated to boiling with 5 ml of ethyl acetate, the mixture is filtered, while hot, through a folded paper filter, and the filtrate is allowed to cool under stirring. After the precipitation of the crystals, the mixture is stirred at 25°C for further 2 hours, the crystals are filtered off, and washed twice using 2 ml of cold ethyl acetate each time. The crude product is recrystallized from 7.5 ml of ethyl acetate.

Thus, 1.15 g (80.7%) of the title compound S-(-)-1-[9'H--carbazol-4'-yloxy] -3- [{2''-(2'''-<methoxy>-phenoxy)-ethyl}--amino]-propan-2-ol are obtained as white crystals. M.p.: 110 to 111.5°C.
[α]_{D} = +18.1° (20°C, c = 1, acetic acid).

### Preparation of the starting compound:

### Preparation of N- [2- (2'-<methoxy>-phenoxy) -ethyl]--benzylamine of formula V

### N- [2- (2'-<methoxy>-phenoxy) -ethyl]--benzylamine dihydrate

To 131 ml (128.64 g, 1.2 moles) of benzylamine heated to 80°C 69.33 g (0.3 moles) of 1-[2'-(bromo)-ethoxy]-2-[methoxy]--benzene are added in 25 minutes. The rate of addition is adjusted to maintain the inner temperature of the reaction mixture at 95 to 105°C. After the addition, the mixture is stirred at an inner temperature of 95 to 100°C for 2 hours, the suspension obtained is cooled in ice-water to 25°C, then poured into 1 000 ml of 10% by weight hydrochloric acid cooled with ice-water in 5 minutes taking care that the inner temperature should not exceed 50°C. The solution obtained is cooled with ice-water. In 5 to 10 minutes, the hydrochloride of the product precipitates in the form of crystals. The crystal suspension is stirred at 5 to 10°C for half an hour, filtered, washed with water. The crude hydrochloride is recrystallized from 400 ml of water, filtered and washed with water.

Thus, 71.9 g (81.6%) of the hydrochloride of the title compound N-[2-(2'-<methoxy>-phenoxy)-ethyl]-benzylamine dihydrate are obtained. M.p.: 148 to 150°C.

The hydrochloride is suspended in 300 ml of water, the suspension is heated until the crystals dissolve, and, to the warm solution, 100 ml of 10% by weight aqueous sodium hydroxide solution are added, drop by drop. The solution is cooled with ice-water to a temperature of 5 to 10°C, the crystals are filtered, washed with 100 ml of cold water and dried on the air at room temperature.

Thus, 43.1 g (48.9%) of the title compound N-[2-(2'--<methoxy>-phenoxy)-ethyl]-benzylamine dihydrate are obtained. The product contains crystal water (C₁₆H₁₉NO₂.2H₂O). M.p.: 53 to 55°C.

### Example for Comparison No. I

### (Reproduction of Example 5 of DE-OS No. 28 15 926)

A mixture of 60.4 g of 4-(oxiranylmethoxy)-9H-carbazole, 64.8 g of N-[2-(2'-<methoxy>-phenoxy)-ethyl]-benzylamine and 200 ml of ethylene glycol dimethyl ether was boiled for 24 hours. On cooling the mixture, the product did not precipitate. The mixture was evaporated under reduced pressure, the residual oil could not be crystallized from ethanol, isopropanol and acetone, wherefore it was transferred to a column filled with silica gel. In the column chromatography 1,2-dichloromethane, a mixture containing 9 volumes of 1,2--dichloroethane and 1 volume of ethyl acetate, a mixture containing 7 volumes of 1,2-dichloroethane and 3 volumes of ethyl acetate, and, at last, ethyl acetate were used as the solvent. The fractions containing the product were combined and evaporated to yield 75 g (60%) of 1-[N-{benzyl}-2'-({2''--<methoxy>-phenoxy)-ethyl}-amino]-3- [9"'H-carbazol-4"'--yloxy]-propan-2-ol <benzyl-carvedilol>. M.p.: 92°C.

### Example for Comparison No. II

A mixture of 1.92 g (8 mmoles) of 4-(oxiranylmethoxy)-9H--carbazole, 3.08 g (10.5 mmoles) of N-[2-(2'-<methoxy>-phenoxy)--ethyl]-benzylamine and 20 ml of ethyl acetate was boiled. The reaction was followed by means of HPLC. After 28 hours, the reaction mixture contained merely 3% by weight of 1-[N-{benzyl}-2'-({2''-<methoxy>-phenoxy)-ethyl}-amino] -3-- [9"'H-carbazol-4"'-yloxy]-propan-2-ol [benzyl-carvedilol], practically the whole amount of the starting 4-(oxiranylmethoxy)-9H-carbazole remained unreacted. The characteristics of HPLC:
Column: LiChrospher 100RP-18 (5 *µ*m), λ = 254 nm.
Acetonitrile: buffer solution = 1 : 1
flow velocity: 0.5 ml/min.
Buffer solution: 7.74 g of ammonium acetate and 10.5 ml of
acetic acid in 1 000 ml of aqueous solution.

### Retention times:

| | |
|---|---|
| N-[2-(2'-<methoxy>-phenoxy)-ethyl]-benzylamine | 2.0 min. |
| 4-(oxiranylmethoxy)-9H-carbazole 1-[N-{benzyl}-2'-({2''-<methoxy>-phenoxy)- | 4.6 min. |
| -ethyl}-amino]-3- [9'''H-carbazol-4'''-yloxy] --propan-2-ol [benzyl-carvedilol] | 14.1 min. |

### Example for Comparison No. III

The procedure of Example for Comparison No. II was repeated with the difference that 20 ml of dioxane were used as the solvent. After 28 hours of reaction, about 50% by weight of the amount of the starting 4-(oxiranylmethoxy)-9H-carbazole was still present as determined by HPLC.

## Claims

1. R-(+)-1-[N-{benzyl}-2'-({2''-<methoxy>-phenoxy)--ethyl}-amino]-3-[9'''H-carbazol-4'''-yloxy]-propan--2-ol and acid addition salts thereof.

2. S-(-)-1-[N-{benzyl}-2'-({2''-<methoxy>-phenoxy)--ethyl}-amino]-3-[9'''H-carbazol-4'''-yloxy]-propan--2-ol and acid addition salts thereof.
